# EUROPEAN PATENT SPECIFICATION

(11) **EP 3 518 741 B1**
(45) Date of publication and mention of the grant of the patent: **16.09.2020**
(21) Application number: 17781290.6
(22) Date of filing: 26.09.2017
(51) Int. Cl.: A61B 5/00, A61B 5/04, A61B 5/042, A61B 18/14

(54) **MAPPING AND/OR ABLATION CATHETER WITH A CLOSED DISTAL LOOP**
KATHETER ZUR ABBILDUNG UND/ODER ZUR ABLATION MIT EINER GESCHLOSSENEN ENDSCHLEIFE
CATHÉTER POUR LA CARTOGRAPHIE ET/OU POUR L'ABLATION AVEC UNE BOUCLE DISTALE CLOSE

(30) Priority: 27.09.2016 US 201662400508 P
(43) Date of publication of application: 07.08.2019
(73) Proprietor: Cardiac Pacemakers, Inc., St. Paul, MN 55112-5798 (US)
(72) Inventor: SHUROS, Allan C., St. Paul Minnesota 55116 (US); KAUPHUSMAN, James, Champlin Minnesota 55316 (US)
(74) Representative: Pfenning, Meinig & Partner mbB
(86) International application number: PCT/US2017/053533
(87) International publication number: WO 2018/064070

(56) References cited:
- US-A1- 2005 107 678
- US-A1- 2007 299 435
- US-A1- 2010 087 848
- US-B1- 6 610 055

## Description

### CROSS-REFERENCE TO RELATED APPLICATION

This application claims priority to Provisional Application No. 62/400,508, filed September 27, 2016.

### TECHNICAL FIELD

The present disclosure generally relates to an apparatus for providing a therapy to a patient. More particularly, the present disclosure relates to a catheter for mapping and ablating tissue within the heart of the patient.

### BACKGROUND

Atrial fibrillation is a condition in the heart causing irregular heartbeats due to generation of abnormal electrical signals. Various treatment regimens may be followed for treating arrhythmias, such as anti-arrhythmic medications and catheter ablation.

Catheter ablation is a minimally invasive procedure that involves killing an abnormal heart muscle responsible for heart racing. This produces a small area of dead heart muscle called a lesion. A catheter used for ablation may be positioned within a patient's heart. Thus, proper positioning and catheter design are important aspects to provide desirable therapy and avoid damage to the patient's heart. The document US 2005/107678 A1 describes an apparatus comprising a catheter and a deployable tip section with electrodes,

### SUMMARY

In Example 1, an apparatus is described for performing at least one of mapping and ablation functions, the apparatus including: a catheter sized and shaped for vascular access and including an elongate body extending between a proximal end and a distal end; a tip section arranged at the distal end of the elongate body having a closed loop; and one or more electrode structures arranged with the closed loop.

In Example 2, the apparatus is described wherein the closed loop comprises an interior surface and an exterior surface, and at least one of the interior surface and the exterior surface is continuous about the closed loop.

In Example 3, the apparatus is described wherein the one or more electrode structures includes a plurality of electrode structures, and the plurality of electrode structures are arranged on the exterior surface of the closed loop.

In Example 4, the apparatus is described wherein each of the plurality of electrode structures comprises a curved portion.

In Example 5, the apparatus is described wherein the exterior surface of the closed loop comprises a curved surface and the interior surface of the closed loop comprises a substantially linear surface, and the curved portion of each of the plurality of electrode structures is arranged on the curved surface of the closed loop.

In Example 6, the apparatus is described wherein the closed loop comprises a substantially circular shape.

In Example 7, the apparatus is described wherein the closed loop includes a deployed configuration and a constricted configuration, and the closed loop is configured to collapse to the constricted configuration in response to constriction thereof.

In Example 8, the apparatus is described further comprising an actuation wire arranged within a lumen of the closed loop, the actuation wire being configured to actuate the closed loop between the deployed configuration and the constricted configuration in response to tensioning of the actuation wire.

In Example 9, the apparatus is described wherein the closed loop is configured to retain the substantially circular shape after being actuated from the constricted configuration to the deployed configuration.

In Example 10, the apparatus is described wherein the closed loop is arranged approximately perpendicular to the elongate body in the deployed configuration.

In Example 11, the apparatus is described wherein the closed loop is configured to self-deploy to the deployed configuration in response to release of the constriction.

In Example 12, the apparatus is described further comprising a memory wire arranged within the lumen of the closed loop, the memory wire being configured to retain the closed loop in the substantially circular shape.

In Example 13, the apparatus is described wherein the actuation wire is further configured to steer the elongate body of the catheter in response to tensioning of the actuation wire.

In Example 14, the apparatus is described wherein the closed loop is configured to maintain the approximately perpendicular arrangement with respect to the elongate body in response to tensioning of the actuation wire.

In Example 15, the apparatus is described wherein one or more electrode structures comprises a plurality of electrode structures, and the plurality of electrode structures are uniformly distributed about the closed loop.

In Example 16, an apparatus is described for performing at least one of mapping and ablation functions, the apparatus comprising: a catheter sized and shaped for vascular access and including an elongate body extending between a proximal end and a distal end; a tip section arranged at the distal end of the elongate body, the tip section including a substantially linear portion and a substantially circular portion forming a closed loop; and one or more electrode structures arranged with the substantially circular portion.

In Example 17, the apparatus is described wherein the closed loop comprises an interior surface and an exterior surface, and at least one of the interior surface and the exterior surface is continuous about the substantially circular portion forming the closed loop.

In Example 18, the apparatus is described wherein the interior surface of the substantially circular portion forming the closed loop comprises an uninterrupted surface.

In Example 19, the apparatus is described wherein the one or more electrode structures includes a plurality of electrode structures, and the plurality of electrode structures are arranged on the exterior surface of the closed loop, and each of the plurality of electrode structures comprise a curved portion.

In Example 20, the apparatus is described wherein the exterior surface of the closed loop comprises a curved surface, and the curved portion of each of the plurality of electrode structures is arranged on the curved surface of the closed loop.

In Example 21, the apparatus is described wherein the closed loop includes a deployed configuration and a constricted configuration, and the closed loop is configured to collapse to the constricted configuration in response to constriction thereof.

In Example 22, the apparatus is described further comprising an actuation wire arranged within a lumen of the closed loop, the actuation wire being configured to actuate the closed loop between the deployed configuration and the constricted configuration in response to tensioning of the actuation wire.

In Example 23, the apparatus is described wherein the closed loop is arranged between at an angle between 60 degrees and a 110 degrees relative to the substantially linear portion.

In Example 24, the apparatus is described further comprising a deployment sheath configured to maintain the closed loop in a delivery configuration.

In Example 25, the apparatus is described wherein the closed loop configured to position approximately perpendicular to the substantially linear portion in response to deployment from the deployment sheath.

In Example 26, a system is described for performing at least one of mapping and ablation functions on a patient, the system comprising: a catheter sized and shaped for vascular access of the patient, the catheter including: an elongate body extending between a proximal end and a distal end, a tip section arranged at the distal end of the elongate body having a closed loop, one or more electrode structures arranged on the closed loop; and an external device configured to determine a position of the catheter.

In Example 27, the system is described wherein the external device is configured to inject current into the patient and measure a corresponding voltage on the one or more electrode structures to determine the position of the catheter in response thereto.

In Example 28, the system is described further comprising one or more navigation sensors arranged with the tip section, and the external device is configured to sense the one or more navigation sensors to determine the position of the catheter.

In Example 29, the system is described wherein the tip section includes a substantially linear portion, and a substantially circular portion forming the closed loop, the one or more navigation sensors comprises a first navigation sensor arranged with the substantially linear portion and a second navigation sensor arranged with the substantially circular portion.

In Example 30, the system is described wherein the external device is configured to sense the first navigation sensor and the second navigation sensor to determine the position of the catheter.

In Example 31, the system is described wherein the closed loop comprises radiopaque properties, and the external device configured to sense the radiopaque properties to determine the position of the catheter.

In Example 32, a method is described comprising: navigating a catheter sized and shaped for vascular access toward a target therapy location of a patient, the catheter including an elongate body extending between a proximal end and a distal end, a tip section arranged at the distal end of the elongate body and having a substantially linear portion and a substantially circular portion forming a closed loop, and one or more electrode structures arranged with the substantially circular portion; arranging the closed loop to at least partially surround the target therapy location; and performing at least one of mapping and ablation functions via the one or more electrode structures at the target therapy location.

In Example 33, the method is described further comprising determining a position of the catheter prior to the performing of at least one of mapping and ablation functions.

In Example 34, the method is described, the catheter further comprising one or more navigation sensors arranged with the tip section, and wherein determining the position of the catheter includes sensing the or more navigation sensors via an external device.

In Example 35, the method is described wherein determining the position of the catheter comprises injecting current into the patient via an external device, and measuring a corresponding voltage on the one or more electrode structures to determine the position of the catheter in response thereto

While multiple embodiments are disclosed, still other embodiments will become apparent to those skilled in the art from the following detailed description, which shows and describes illustrative embodiments. Accordingly, the drawings and detailed description are to be regarded as illustrative in nature.

### BRIEF DESCRIPTION OF THE DRAWINGS

FIG. 1 shows an exemplary ablation system in accordance with embodiments of the disclosure.
FIG. 2 shows another exemplary catheter in accordance with embodiments of the disclosure.
FIG. 3 shows another exemplary catheter in accordance with embodiments of the disclosure.
FIG. 4A shows a partial cut-away illustration of a catheter apparatus for performing at least one of mapping and ablation functions in accordance with embodiments of the disclosure.
FIG. 4B shows a cross-sectional view of a portion of the catheter apparatus shown in FIG. 4A in accordance with embodiments of the disclosure.
FIG. 5A shows an exemplary deployment sheath and a catheter therein in accordance with embodiments of the disclosure.
FIG. 5B shows the catheter shown in FIG. 5A in a deployed configuration as deployed from the deployment sheath in accordance with embodiments of the disclosure.
FIG. 6 shows a portion of an exemplary telescoping tip section of a catheter in accordance with embodiments of the disclosure.
FIG. 7 shows an exemplary system for performing at least one of mapping and ablation functions on a patient in accordance with embodiments of the disclosure.

While the apparatus is amenable to various modifications and alternative forms, specific embodiments have been shown by way of example in the drawings and are described in detail below.

### DETAILED DESCRIPTION

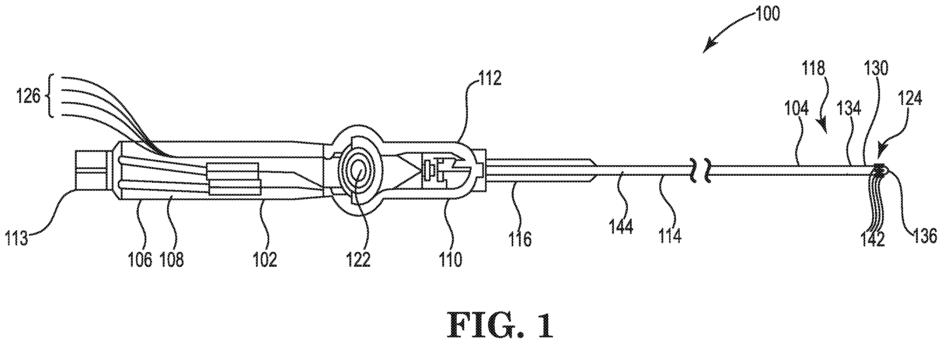

FIG. 1 shows an exemplary ablation system 100 in accordance with embodiments of the disclosure. As shown, the system 100 includes a catheter 102 sized and shaped for vascular access. The catheter 102 has a distal end 104 and a proximal end 106. In one aspect, the proximal end 106 of the catheter 102 includes a handle 108 having a proximal portion 110 and a distal portion 112, and configured to be manipulated by a physician during a diagnostic and/or treatment procedure. The handle 108 may include a plurality of conduits, conductors, and wires to facilitate control of the catheter 102 and/or mating of the catheter 102 with a source of fluid, a source of ablative energy, a source of mapping, temperature display, sensors, and/or control software/hardware. The handle 108 further includes a connection port 113 through which ablative energy source and a mapping energy source may be operably coupled.

The catheter 102 may include an elongate body 114 having a proximal end 116 and a distal end 118. The elongate body 114 may house electrical conductors/ cable assembly (e.g., wires) for transmitting sensed signals and/or ablation energy. In addition, the elongate body 114 may include a circular cross-sectional geometry. However, other cross-sectional shapes, such as elliptical, rectangular, triangular, and various other shapes, may be provided. In certain instances, the elongate body 114 may be preformed of an inert, resilient material that retains its shape and does not soften significantly at body temperature; for example, Pebax®, polyethylene, or Hytrel® (polyester). The elongate body 114 may be made of a variety of materials, including, but not limited to, metals and polymers. The elongate body 114 may be flexible and capable of winding through a tortuous path that leads to a target site, i.e., an area within the heart. The elongate body 114 may also be semi-rigid, i.e., by being made of a stiff material, or by being reinforced with a coating or coil, to limit the amount of flexing.

In certain instances, the movement of the distal end 118 of the elongate body 114 (such as to wind through the tortuous path that leads to a target site) may be controlled by a control mechanism 122 included within the handle 108. The system 100 may include an articulating section of the elongate body 114 (e.g., near the distal end 118) that is controlled via the control mechanism 122. The distal end 118 of the elongate body 114 may be deflected or bent. The articulation section of the body may facilitate insertion of the catheter 102 through a body lumen (e.g., vasculature) and/or placement of electrodes at a target tissue location. The articulation may provide one or more degrees of freedom and permit up/down and/or left/right articulation.

The distal end 104 of the catheter 102 includes a tip section 124 positioned at the distal end 118 of the elongate body 114. The tip section 124 includes a proximal portion 134 and a distal portion 136. In certain instances, portions of the tip section 124 may be formed from a conductive material. More specifically, the system 100 includes one or more electrode structures 142, formed of the conductive material, on an exterior surface 130 of the tip section 124. The electrode structures 142 may be arranged around a circumference of exterior surface 130 of the tip section 124. In addition, the electrode structures 142 may be configured as mapping electrodes and ablation electrodes.

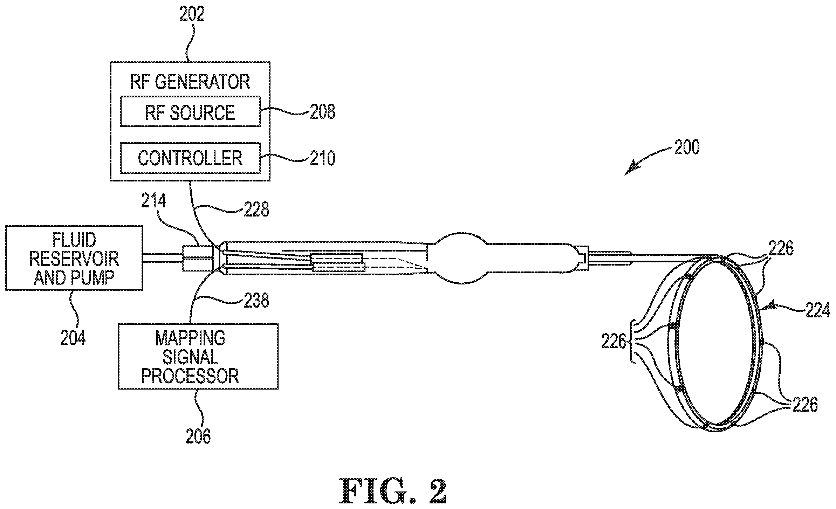

In various embodiments, the electrode structures 142 may be configured to conduct radio frequency (RF) energy used to form lesions during the ablation procedure. The electrode structures 142 may deliver ablation energy to the myocardial tissues that are the source of arrhythmia, thereby destroying them or a portion thereof through heat. In certain instances, the electrode structures 142 may be electrically coupled to an RF generator, which will be discussed in further detail with reference to FIG. 2. The ablation energy may be conveyed from the RF generator to the electrode structures 142 to form localized lesions in the myocardial tissues. Each of the electrode structures 142 may be coupled to wires 126 using suitable means, such as soldering or welding. The number of wires 126 may be equal to the number of electrode structures 142. The wires 126 may pass through a lumen 144 extending through the elongate body 114 of the catheter 102, where it is further electrically coupled to the cable assembly 120 located within the handle 108 and to the RF generator exteriorly coupled to the ablation system 100.

The electrode structures 142 may also be configured to measure the localized intracardial electrical activity in real time at the point of RF energy delivery. The electrode structures 142 allow the physician to ascertain lesion formation by measuring the electrical activity of the tissue having been in contact with an ablation electrode (e.g., the lack of electrical activity indicates ablated tissue, whereas the presence of electrical activity indicates live tissue). In certain instances, the wires 126, coupled to the electrode structures 142, may also be electrically coupled to a mapping signal processor, which will be discussed in further detail with regards to FIG. 2, so that electrical events in myocardial tissue may be sensed for the generation of electrograms, monophasic action potentials (MAPs) and isochronal electrical activity maps.

In some embodiments, the electrode structures 142 may be operable solely for sensing the localized intracardial electrical activity for mapping the target cardiac structure (e.g., the pulmonary vein ostia) prior to or during a pulmonary vein isolation procedure. In such embodiments, the catheter 102 lacks ablation capabilities, and the actual ablation energy is delivered by a separate catheter / system.

FIG. 2 shows another exemplary ablation system in accordance with embodiments of the disclosure. The ablation system may include cooling, ablation, and mapping system components and a catheter 200. The catheter 200 may be configured to be introduced through the vasculature of the patient, and into one of the chambers of the heart, where it may be used to map and/or ablate myocardial tissue. The catheter 200 also comprises a radio frequency (RF) generator 202, a fluid reservoir, a pump 204, a mapping signal processor 206, coupled to the catheter 200 via a cable assembly or through connection port 214. In certain instances, the radio frequency (RF) generator 202, and the mapping signal processor 206 may be connected to the catheter 200 through wires 228, 238. The fluid reservoir and pump 204 may be connected to the catheter 200 through the connection port 214. Although the radio frequency (RF) generator 202, the fluid reservoir and the pump 204, and the mapping signal processor 206 are shown as discrete components, these components may alternatively be incorporated into a single integrated device.

The catheter 200 may include a tip section 224 with a plurality of electrode structures 226 (e.g., 10 - 20 electrode structures) arranged. The tip section 224 may form a closed loop. One or more of the plurality of electrode structures 226 may be operably connected to the RF generator 202. The RF generator 202 may be used to generate the energy for the ablation procedure. The RF generator 202 includes an RF source 208 for the RF energy and a controller 210 for controlling the timing and the level of the RF energy delivered through the plurality of electrode structures 226. The illustrated catheter 200 also includes the fluid reservoir and pump 204 for pumping cooling fluid, such as a saline, through an inner fluid lumen of the catheter 200 to the tip section 224. As ablation energy moves through the tip section 224, areas of increased current density may develop and result in localized hotspots. The catheter 200 may reduce the effect of the proximal end by pumping cooling fluid to the tip section 224. The cooling fluid may heat up as a result of the hot spots. Thus, the pump 204 may also return the heated fluid through the catheter 200 for egress from the catheter 200 to a proximal location, such as the fluid reservoir and pump 204 for cooling and/or recirculation.

The mapping signal processor 206 may be operably coupled to the one or more of the plurality of electrode structures 226. The mapping signal processor 206 may be configured to detect, process, and record electrical signals within the heart via the one or more of the plurality of electrode structures 226. Based on the electrical signals sensed by the one or more electrodes, the physician may identify the specific target tissue sites within the heart, and ensure that the arrhythmia causing substrates have been electrically isolated by the ablative treatment. Based on the detected electrical signals, the mapping signal processor 206 outputs electrocardiograms (ECGs) to a display (not shown), which may be analyzed by the physician to determine the existence and/or location of arrhythmia substrates within the heart and/or determine the location of the catheter 200 within the heart. In certain instances, the mapping signal processor 206 may generate an isochronal map of the detected electrical activity and output the map to the display for analysis by the physician.

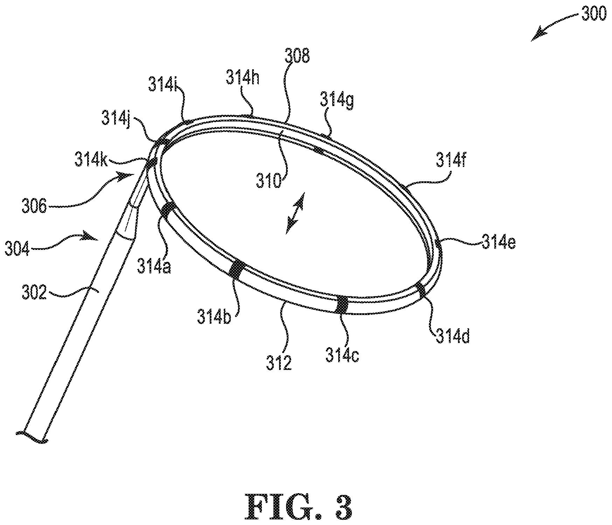

FIG. 3 shows another exemplary catheter 300 in accordance with embodiments of the disclosure. The catheter 300 may be configured to perform at least one of mapping and ablation functions (e.g. as discussed above with reference to FIG. 1 and FIG. 2). The catheter 300, sized and shaped for vascular access, may include an elongate body 302 that extends between a proximal end (not shown) and a distal end 304 of the catheter 300. The proximal end of the elongate body 302 may be coupled to a handle assembly (e.g. as discussed above with reference to FIG. 1 and FIG. 2). The catheter 300 may also include a tip section 306 arranged at the distal end 304 of the elongate body 302. The tip section 306 may form a closed loop 308.

The closed loop 308 forms a continuous structure. As shown in FIG. 3, the closed loop 308 is a substantially circular structure. The closed loop 308 does not include a free tip (e.g., a tip that is unattached to some other structure) or open end. Thus, the closed loop 308 is a gapless structure arranged at the distal end 304 of the elongate body 302. The closed loop 308 may provide atraumatic benefits to the catheter 300. The closed loop 308 being an endless or gapless structure may avoid puncturing, catching on, or otherwise entangling with portions of a patient's heart during an ablation procedure. More specifically, the catheter 300 may be moved into position and be repositioned as part of the ablation procedure. The closed loop 308, having no ends, will avoid catching on aspects of the portions of the patient's heart such as a valve. In addition, the closed loop 308 may at least partially surround a target ablation area. In positioning the closed loop 308 to the target ablation area, the closed loop 308 may facilitate avoiding traumatic contact with the heart wall due to the continuous/endless structure of the closed loop 308.

As shown in FIG. 3, the closed loop 308 may include an interior surface 310 and an exterior surface 312. To form the closed loop 308, at least one of the interior surface 310 and the exterior surface 312 are continuous about the closed loop 308. The closed loop 308 may also include one or more electrode structures 314a-k. The electrode structures 314a-k may be configured 2. to provide RF energy used to form lesions during an ablation procedure. In addition, the electrode structures 314a-k may also be configured to measure the localized intracardial electrical activity (map) in real time at the point of RF energy delivery (as described in further detail above with reference to FIG. 1 and FIG. 2).

The electrode structures 314a-k may be arranged about a circumference of the closed loop 308, and arranged on the exterior surface 312 of the closed loop 308. The closed loop 308 may be formed of an insulative material in order to electrically isolate the electrode structures 314a-k from one another. In addition, with the electrode structures 314a-k being arranged on the exterior surface 312, the closed loop 308 may electrically isolate the electrode structures 314a-k from the internal portion of the closed loop 308. More specifically, energy resulting from activated ones of the electrode structures 314a-k is directed externally from the closed loop 308 with the energy being isolated from the internal portion of the closed loop 308.

Any one or more of the components depicted in any of the FIG. 3 may be, in embodiments, integrated with various other components depicted therein (and/or components not illustrated). For example, the closed loop 308 and electrode structures 314a-k may be used in connection with ablation system 100 and catheter 200.

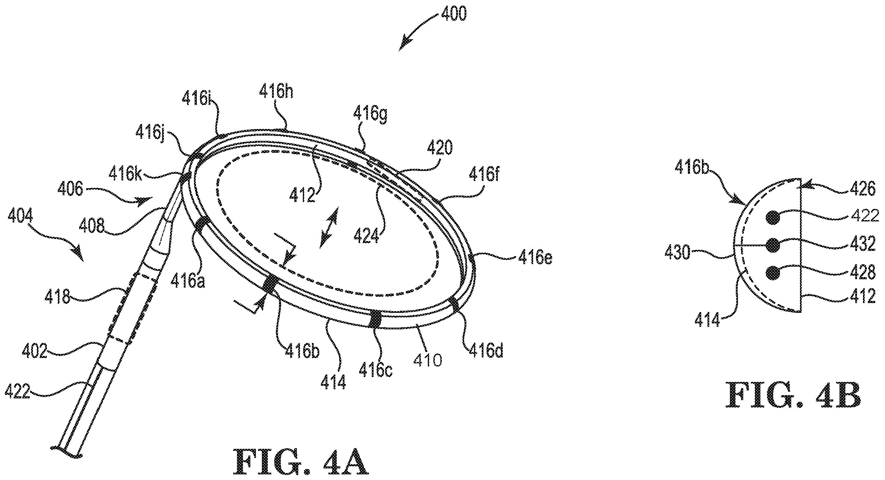

FIG. 4A shows a partial cut-away illustration of an apparatus for performing at least one of mapping and/or ablation functions in accordance with embodiments of the disclosure. The apparatus may be a catheter 400 sized and shaped for vascular access having an elongate body 402. The elongate body 402 extends between a proximal end (not shown) and a distal end 404. The proximal end of the elongate body 402 may be coupled to a handle assembly (e.g. as discussed above with reference to FIG. 1 and FIG. 2). The catheter 400 may also include a tip section 406 arranged at the distal end 404 of the elongate body 402.

The tip section 406 may include a substantially linear portion 408 and a substantially circular portion 410 that forms a closed loop. The substantially circular portion 410 may include different shapes including an oval shape, elliptical shape, or other similar curved shape. The substantially circular portion 410 may be arranged at an angle relative to the substantially linear portion 408. More specifically, the substantially circular portion 410 may be arranged at an angle between 60 degrees and a 110 degrees relative to the substantially linear portion 408. As shown in FIG. 4A, the substantially circular portion 410 is arranged approximately perpendicular to the substantially linear portion 408. The substantially circular portion 410 is continuous such that the substantially circular portion 410 forms a gapless structure as the substantially circular portion 410 forms a closed loop that extends from the substantially linear portion 408.

In addition, the substantially circular portion 410 may include an interior surface 412 and an exterior surface 414. As shown in FIG. 4A, the interior surface 412 may be a substantially linear surface and the exterior surface 414 may be a curved surface. The interior surface 412 having a substantially linear surface and the exterior surface 414 having a curved surface may enhance the ability of the substantially circular portion 410 to collapse. The substantially circular portion 410 may be differently sized than the elongate body 402. The substantially circular portion 410 have less of a thickness than a thickness of the elongate body 402. More specifically, the substantially circular portion 410 may be between 1 and 1.67 mm (3 and 5 French) and the elongate body 402 may be between 1.67 and 2.67 mm (5 5 and 8 French). In addition, the substantially circular portion 410 may have a greater flexibility than the elongate body 402. The substantially circular portion 410 may be formed of an insulative material (e.g., low durometer Pebax®) and the elongate body 402 may be Pebax®, polyethylene (Low or high density), or polyurethane. The substantially linear portion 408 may also be similarly sized and formed from a similar material as the substantially circular portion 410.

The substantially circular portion 410 may also include a plurality of electrode structures 416a-k. In embodiments in which the catheter 400 is configured to perform an ablation procedure in addition to mapping, the plurality of electrode structures 416a-k may be configured to deliver the RF energy used to form lesions during the ablation procedure. In addition, the electrode structures 416a-k may also be configured to measure the localized intracardial electrical activity (map) in real time at the point of RF energy delivery (as described in further detail above with reference to FIG. 1 and FIG. 2). The plurality of electrode structures may be arranged uniformly about a circumference of the substantially circular portion 410, and arranged on the exterior surface 414. The substantially circular portion 410 may be formed of an insulative material in order to electrically isolate the electrode structures 416a-k from one another. In addition, with the plurality of electrode structures 416a-k being arranged on the exterior surface 414, the substantially circular portion 410 may electrically isolate the plurality of electrode structures 416a-k from the internal portion of the substantially circular portion 410. More specifically, energy resulting from activated ones of the plurality of electrode structures 416a-k is directed externally from the substantially circular portion 410 with the energy being isolated from the internal portion of the substantially circular portion 410.

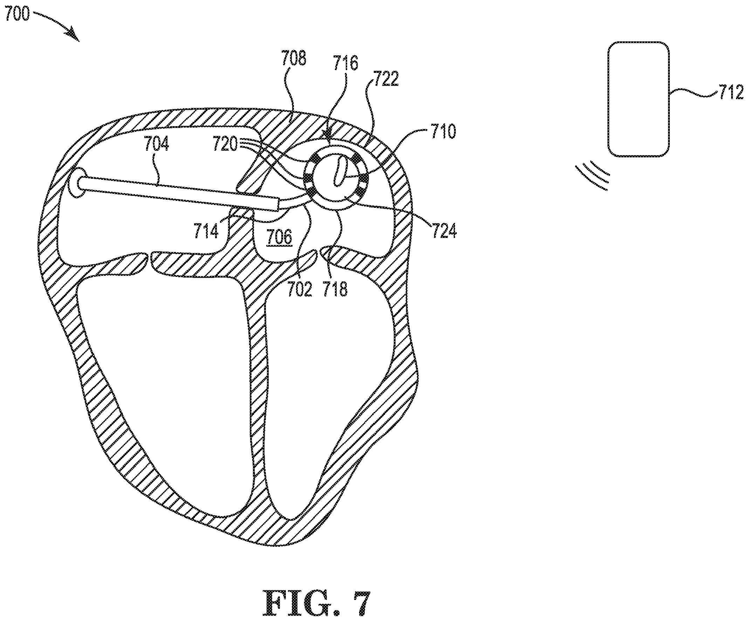

The catheter 400 may also optionally include or more navigation sensors arranged with the tip section 406. In certain instances, a first navigation sensor 418 may be arranged with the substantially linear portion 408, and a second navigation sensor 420 arranged with the substantially circular portion 410. The second navigation sensor 420 may be arranged at any location about the circumference of the substantially circular portion 410. In addition, the first navigation sensor 418 and the second navigation sensor 420 may be arranged within a lumen of the substantially linear portion 408 and the substantially circular portion 410 respectively. As discussed in further detail below with reference to FIG. 7, an external device (not shown) may be configured to sense one or more of the first navigation sensor 418 and the second navigation sensor 420 to determine a position of the catheter 400. In addition, the substantially circular portion 410 may also include radiopaque properties (e.g., barium sulfate injected into the material of the substantially circular portion 410). The external device may also be configured to sense the radiopaque properties to determine the position of the catheter 400.

In certain instances, the substantially (closed loop) circular portion 410 may be configured to collapse to a constricted configuration 424 in response to constriction of the substantially (closed loop) circular portion 410. The catheter 400 may include at least one actuation wire 422 that extends within a lumen of the elongate body 402. The at least one actuation wire 422 may also be arranged within a lumen of the substantially (closed loop) circular portion 410 (shown in FIG. 4B). The at least one actuation wire 422 may be configured to actuate the substantially circular portion 410 between a deployed configuration (shown in FIG. 4A) and the constricted configuration 424 and in response to tensioning of the at least one actuation wire 422. The substantially (closed loop) circular portion 410 may be configured to retain the substantially circular shape after being in the constricted configuration 424. More specifically, the substantially (closed loop) circular portion 410 is configured to redeploy to the deployed configuration after release of tension of the at least one actuation wire 422. Further, the substantially (closed loop) circular portion 410 is configured to maintain an approximately perpendicular arrangement, relative to the substantially linear portion 408, in the constricted configuration 424, and also redeploy to the approximately perpendicular arrangement after release of tension of the at least one actuation wire 422.

The substantially (closed loop) circular portion 410 may be suited for mapping and/or ablation procedures in endocardial sites, such as, for example, within a blood vessel, such as a pulmonary vein, or an ostium of a blood vessel, such as the ostium of a pulmonary vein. For example, adjustable nature of the substantially (closed loop) circular portion 410 and the arrangement of the substantially (closed loop) circular portion 410 relative to the elongate body may allow for the substantially (closed loop) circular portion 410 to press tight against the endocardial site. This may be useful in both mapping and ablation procedures, and enhance the positioning of the substantially (closed loop) circular portion 410 relative to the endocardial site, or to adjust to endocardial sites of different diameters (e.g. that of an adult or large animal, or a small child or small animal), or both. This ability to adjust the substantially (closed loop) circular portion 410 may also be advantageous in mapping procedures to better localize the source of the cardiac arrhythmia, and may be used in ablation procedures to focus the ablation energy on the selected endocardial site. In addition, the substantially (closed loop) circular portion 410 being a continuous structure may provide complete circumferential coverage of the endocardial site. This may allow for three dimensional mapping of the endocardial site.

In certain instances, the at least one actuation wire 422 may also be configured to steer the elongate body 402 of the catheter 400 in response to tensioning of the least one actuation wire 422. In other instances, the least one actuation wire 422 includes a dedicated actuation wire for steering the elongate body 402 and a dedicated actuation wire for constricting the substantially (closed loop) circular portion 410.

FIG. 4B shows a cross-sectional view of a portion of the catheter 400 shown in FIG. 4A in accordance with embodiments of the disclosure. The cross-sectional view shown in FIG. 4B is taken from the line shown in FIG. 4A. A lumen 426 of the substantially (closed loop) circular portion 410 may house a number of different wires. For illustrative purposes, the wires are shown linearly and separate, however, the wires may be bundled and/or arranged centrally within the lumen 426. The at least one actuation wire 422 may be arranged within the lumen 426 about a circumference of the substantially (closed loop) circular portion 410. The at least one actuation wire 422 may loop around within the lumen 426 such that tension of the at least one actuation wire 422 constricts the substantially (closed loop) circular portion 410 to the constricted configuration 424 shown in FIG. 4A.

The lumen 426 may house a memory wire 428 arranged within the lumen 426 of the substantially (closed loop) circular portion 410. The memory wire 428 may be configured to retain the substantially (closed loop) circular portion 410 in the substantially circular shape. The memory wire 428 may be formed from Nitinol or another shape memory material. In addition, the lumen 426 may house a bundle of conductive fibers 432 that are used to couple each of the plurality of electrode structures 416a-k to an RF generator and/or mapping signal processor (e.g., as discussed above with reference to FIG. 2).

One electrode structure 416b of the plurality of electrode structures 416a-k is highlighted in FIG. 4A. As shown in FIG. 4B, the electrode structure 416b includes a curved portion 430. Each of the plurality of electrode structures 416a-k may include a curved portion. The curved portion 430 may be arranged on the exterior surface 414 of the substantially (closed loop) circular portion 410. In certain instances, the curved portion 430 of each of the plurality of electrode structures 416a-k may be formed by printing the plurality of electrode structures 416a-k on the exterior surface 414 of the substantially (closed loop) circular portion 410. In other instances, the plurality of electrode structures 416a-k may be solid structures that are hemispherical with the curved portion 430 as a front surface, and a linear portion as a back surface.

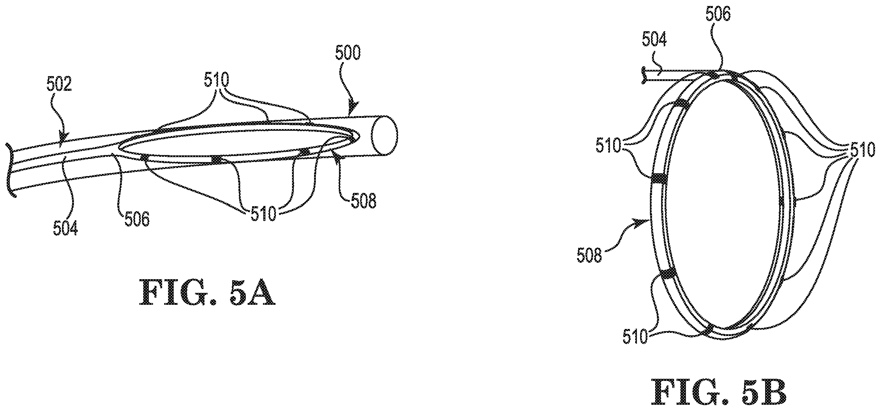

FIG. 5A shows an exemplary deployment sheath 500 and a catheter 502 therein in accordance with embodiments of the disclosure. The deployment sheath 500 may be used to deliver the catheter 502 to a target therapy location. The target therapy location may be one of a number of endocardial sites, such as, for example, within a blood vessel, such as a pulmonary vein, or an ostium of a blood vessel, such as the ostium of a pulmonary vein. The deployment sheath 500 may be configured to maintain the catheter 502 in a constricted/delivery configuration in delivering the catheter 502 to the target therapy location.

The catheter 502 may be configured to perform at least one of mapping and ablation functions (e.g. as discussed above with reference to FIG. 1 and FIG. 2). The catheter 502, sized and shaped for vascular access, may include an elongate body 504 and a tip section 506. The tip section 506 may form a closed loop 508. The closed loop 508 is a continuous structure without gaps about a circumference of the closed loop 508. The closed loop 508 is a substantially circular, oval, oblong, or any other similar structure. The closed loop 508 may also include a plurality of electrode structures 510. The plurality of electrode structures 510 may be configured to provide RF energy used to form lesions during an ablation procedure. In addition, the plurality of electrode structures 510 may also be configured to measure the localized intracardial electrical activity (map) in real time at the point of RF energy delivery (as described in further detail above with reference to FIG. 1 and FIG. 2).

The plurality of electrode structures 510 may be arranged uniformly about a circumference of the closed loop 508. In addition, the plurality of electrode structures 510 may have a hemispherical shape with a curved outer surface and a substantially linear inner surface. As described in detail above with reference to FIGs. 4A-B, the closed loop 508 may similarly have a curved outer surface and a substantially linear inner surface in certain instances. As a result, of these structures, pairs of the plurality of electrode structures 510 on opposite sides the closed loop 508 may collapse toward one another in the constricted/delivery configuration shown in FIG. 5A. In the constricted/delivery configuration, the interior surface of the closed loop 508 on either side thereof may contact such that the opposing pairs of the plurality of electrode structures 510 form a complete circle in effect. The hemispherical structure of the plurality of electrode structures 510 and the hemispherical structure of the closed loop 508, used alone or in combination, may enhance the ability of the closed loop 508 to collapse to the delivery or constricted configuration.

The closed loop 508 is configured to collapse to the constricted/delivery configuration shown in FIG. 5A in response to restriction of the closed loop 508 within the deployment sheath 500. The deployment sheath 500 is configured to maintain the closed loop 508 in the constricted/delivery configuration.

FIG. 5B shows the catheter 502 shown in FIG. 5A in a deployed configuration as deployed from the deployment sheath 500 in accordance with embodiments of the disclosure. In the delivery configuration, the closed loop 508 may be arranged at an angle relative to the elongate body 504. More specifically, the closed loop 508 may be arranged at an angle between 60 degrees and about 110 degrees relative to the elongate body 504. As shown in FIG. 5B, the closed loop 508 is arranged approximately perpendicular to the elongate body 504.

The closed loop 508 is configured to assume the angled position after release from the deployment sheath 500 into the delivery configuration shown in FIG. 5B. The closed loop 508 may include a shape memory material or a shape memory wire within the closed loop 508 that is configured to retain the closed loop 508 in the intended shape and at the intended angle relative to elongate body 504 the after release from the deployment sheath 500.

Any one or more of the components depicted in any of the FIG. 5 may be, in embodiments, integrated with various other components depicted therein (and/or components not illustrated). For example, the deployment sheath 500 may used in connection with ablation system 100, ablation catheter 200, catheter 300, catheter 400, telescoping tip section 600, or system 700.

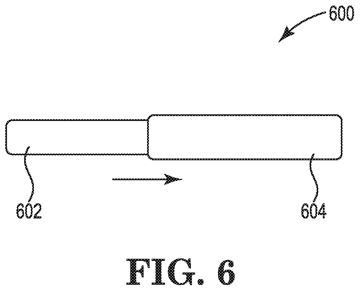

FIG. 6 shows a portion of an exemplary telescoping tip section 600 of a catheter in accordance with embodiments of the disclosure. Although only a portion is shown in FIG. 6, the tip section 600 may be a closed loop structure (e.g., as is discussed in detail above). The tip section 600 may include a first diameter 602 and a second diameter 604, with the second diameter 604 being greater than the first diameter 602. The tip section 600 may have a taper between the first diameter 602 and the second diameter 604.

The tip section 600 may be configured to telescope such that the portion of the tip section 600 having the first diameter 602 constricts the against portion of the tip section having the second diameter 604. In certain instances, the tip section 600 may be configured to telescope such that the portion of the tip section 600 having the first diameter 602 extends within the portion of the tip section having the second diameter 604. The tip section 600 may include an actuation wire (e.g., as discussed above with reference to FIGs. 4A-B), which may be configured to telescope the tip section 600 in response to tensioning of the actuation wire.

FIG. 7 shows an exemplary system 700 for performing at least one of mapping and ablation functions on a patient in accordance with embodiments of the disclosure. The system 700 includes a catheter 702 and an external device 712. A deployment sheath 704 may be navigated into the left atrium 706 of the heart 708 of the patient. The deployment sheath 704 is arranged adjacent to a target therapy location 724. The deployment sheath 704 may be navigated within the left atrium 706 may be accomplished using a vascular introducer retrograde through the aortic and mitral valves, or may use a transseptal approach from the right atrium. A guide wire (not shown) may be used in association with the deployment sheath 704 to aid in directing the deployment sheath 704 through the appropriate artery toward the heart 708.

After the deployment sheath 704 is navigated to and arranged adjacent to the target therapy location 724, the catheter 702 may be placed. The catheter 702 may be navigated into the left atrium 706 of the heart 708 through the deployment sheath 704, and the catheter 702 may be arranged adjacent to the target therapy location 724. A steering mechanism located on with a handle assembly (e.g., discussed in FIG. 1) may be manipulated to place the catheter 702 into firm contact with the endocardial tissue 722 at a perpendicular angle to the wall of the heart 708.

The catheter 702 includes an elongate body 714 that extends and a tip section 716 arranged at a distal end of the elongate body 714. The tip section 716 may form a closed loop 718. The closed loop 718 may include different shapes including an oval shape, elliptical shape, or other similar curved shape. The closed loop 718 may be arranged at an angle relative to the elongate body 714. More specifically, the closed loop 718 may be arranged approximately perpendicular to the elongate body 714. The closed loop 718 is continuous such that the closed loop 718 forms a gapless structure.

The closed loop 718 may also include a plurality of electrode structures 720. The plurality of electrode structures 720 may be configured RF energy used to form lesions during an ablation procedure. In addition, the electrode structures 720 may also be configured to measure the localized intracardial electrical activity (map) in real time at the point of RF energy delivery (as described in further detail above with reference to FIG. 1 and FIG. 2). The plurality of electrode structures may be arranged uniformly about a circumference of the closed loop 718.

To assist in positioning the catheter 702 and the closed loop 718, the external device 712 may be used. The external device 712 may be configured to determine a position of the catheter 702. In certain instances, the external device 712 may be configured to inject current into the patient and measure a corresponding voltage on the plurality of electrode structures 720 to determine the position of the catheter 702 in response thereto.

In addition, the catheter 702 optionally may include one or more navigation sensors arranged with the tip section 716. In certain instances, a first navigation sensor may be arranged with the elongate body 714, and a second navigation sensor arranged with the closed loop 718. The external device 712 may be configured to sense one or more of the first navigation sensor and the second navigation sensor to determine a position of the catheter 702. In addition, the closed loop 718 may also include radiopaque properties (e.g., barium sulfate injected into the material of the closed loop 718). The external device 712 may also be configured to sense the radiopaque properties to determine the position of the catheter 702. Determining the position of the catheter 702 may occur prior to performing mapping and/or ablation functions via the plurality of electrode structures 720.

The closed loop 718 may be arranged firmly and stably in contact with the endocardial tissue 722. As shown in FIG. 7, the closed loop 718 is arranged at and surrounds the target therapy location 724. A mapping function may then be applied via the plurality of electrode structures 720. A mapping signal processor (e.g., shown in FIG. 2) may be operated in order to obtain and record signals from the myocardial tissue via bipolar pairs of the plurality of electrode structures 720. These signal measurements may be repeated at different locations within the left atrium 706 to ascertain one or more target sites to be ablated. The user may analyze the in a standard manner, or if electrical activity isochronal maps (whether or not combined with anatomical maps), may analyze these, to ascertain these target sites.

In various embodiments, an ablation function may also be provided via the plurality of electrode structures 720. Once the target therapy location 724 has been identified via analysis of the signals or isochronal electrical activity maps, the plurality of electrode structures 720 are placed into firm contact with the target therapy location 724, and the RF generator (e.g., shown in FIG. 2) is then operated in order to convey RF energy to one or more of the plurality of electrode structures 720 (either in the monopolar or bipolar mode), thereby creating a lesion 710.

Various modifications and additions may be made to the exemplary embodiments discussed. The invention is defined by the appended claims.

## Claims

1. An apparatus for performing at least one of mapping and ablation functions, the apparatus comprising:
a catheter (400) sized and shaped for vascular access and including an elongate body (402) extending between a proximal end and a distal end;
a tip section (406) arranged at the distal end of the elongate body (402) and deployable to form a closed loop, the closed loop comprises an interior surface (412) and an exterior surface (414), wherein the exterior surface (414) comprises a curved surface; and
one or more electrode structures (416a-k) arranged with the tip section (406), **characterized in that** the interior surface (412) comprises a surface defining an interior cylindrical space with an axis perpendicular to the plane of the closed loop.

2. The apparatus of claim 1, wherein at least one of the interior surface (412) and the exterior surface (414) is continuous about the closed loop.

3. The apparatus of claim 2, wherein the one or more electrode structures (416a-k) includes a plurality of electrode structures, and the plurality of electrode structures are arranged on the exterior surface (414) of the closed loop.

4. The apparatus of claim 3, wherein each of the plurality of electrode structures (416a-k) comprise a curved portion.

5. The apparatus of claim 4, wherein a curved portion of each of the plurality of electrode structures (416a-k) is arranged on the curved surface of the closed loop.

6. The apparatus of any of claims 1-5, wherein the closed loop comprises a substantially circular shape.

7. The apparatus of claim 6, wherein the closed loop includes a deployed configuration and a constricted configuration, and the closed loop is configured to collapse to the constricted configuration in response to constriction thereof.

8. The apparatus of claim 7, further comprising an actuation wire (422) arranged within a lumen of the closed loop, the actuation wire (422) being configured to actuate the closed loop between the deployed configuration and the constricted configuration in response to tensioning of the actuation wire (422).

9. The apparatus of claim 8, wherein the closed loop is configured to retain the substantially circular shape after being actuated from the constricted configuration to the deployed configuration.

10. The apparatus of any of claims 8 or 9, wherein the closed loop is arranged approximately perpendicular to the elongate body (402) in the deployed configuration.

11. The apparatus of any of claims 8-10, wherein the closed loop is configured to self-deploy to the deployed configuration in response to release of the constriction.

12. The apparatus of any of claims 8-11, further comprising a memory wire (428) arranged within the lumen of the closed loop, the memory wire (428) being configured to retain the closed loop in the substantially circular shape.

13. The apparatus of any of claims 8-12, wherein the actuation wire is further configured to steer the elongate body (402) of the catheter (400) in response to tensioning of the actuation wire.

14. The apparatus of claim 13, wherein the closed loop is configured to maintain the approximately perpendicular arrangement with respect to the elongate body (402) in response to tensioning of the actuation wire.

15. The apparatus of any of claims 1-14, wherein one or more electrode structures (416a-k) comprises a plurality of electrode structures, and the plurality of electrode structures are uniformly distributed about the closed loop.

## Patentansprüche

1. Vorrichtung zur Ausführung mindestens einer aus Abbildungs- und Ablationsfunktion, wobei die Vorrichtung umfasst:
einen Katheter (400), dimensioniert und geformt für einen vaskulären Zugang und aufweisend einen länglichen Körper (402), sich erstreckend zwischen einem proximalen Ende und einem distalen Ende;
einen Spitzenabschnitt (406), angeordnet an dem distalen Ende des länglichen Körpers (402) und ausbreitbar, um eine geschlossene Schleife auszubilden, wobei die geschlossene Schleife eine Innenfläche (412) und eine Außenfläche (414) umfasst, wobei die Außenfläche (414) eine gewölbte Fläche umfasst;
eine oder mehrere Elektrodenstrukturen (416a-k), angeordnet mit dem Spitzenabschnitt (406), **dadurch gekennzeichnet, dass** die Innenfläche (412) eine Fläche umfasst, die einen inneren zylindrischen Raum mit einer Achse senkrecht zu der Ebene der geschlossenen Schleife definiert.

2. Vorrichtung nach Anspruch 1, wobei mindestens eine aus der Innenfläche (412) und der Außenfläche (414) kontinuierlich um die geschlossene Schleife ist.

3. Vorrichtung nach Anspruch 2, wobei die eine oder mehrere Elektrodenstrukturen (416a-k) eine Vielzahl von Elektrodenstrukturen umfasst und die Vielzahl von Elektrodenstrukturen auf der Außenfläche (414) der geschlossenen Schleife angeordnet sind.

4. Vorrichtung nach Anspruch 3, wobei jede von der Vielzahl von Elektrodenstrukturen (416a-k) einen gewölbten Abschnitt umfasst.

5. Vorrichtung nach Anspruch 4, wobei ein gewölbter Abschnitt von jeder von der Vielzahl von Elektrodenstrukturen (416a-k) auf der gewölbten Fläche der geschlossenen Schleife angeordnet ist.

6. Vorrichtung nach einem der Ansprüche 1-5, wobei die geschlossene Schleife eine im Wesentlichen kreisrunde Form umfasst.

7. Vorrichtung nach Anspruch 6, wobei die geschlossene Schleife eine ausgebreitete Konfiguration und eine zusammengezogene Konfiguration aufweist und die geschlossene Schleife dazu ausgestaltet ist, in Reaktion auf Zusammenziehung hiervon in die zusammengezogene Konfiguration zusammenzufallen.

8. Vorrichtung nach Anspruch 7, ferner umfassend einen Betätigungsdraht (422), angeordnet innerhalb eines Lumens der geschlossenen Schleife, wobei der Betätigungsdraht (422) dazu ausgestaltet ist, in Reaktion auf ein Spannen des Betätigungsdrahtes (422) die geschlossene Schleife zwischen der ausgebreiteten Konfiguration und der zusammengezogenen Konfiguration zu betätigen.

9. Vorrichtung nach Anspruch 8, wobei die geschlossene Schleife dazu ausgestaltet ist, die im Wesentlichen kreisrunde Form beizubehalten, nachdem sie von der zusammengezogenen Konfiguration in die ausgebreitete Konfiguration betätigt wurde.

10. Vorrichtung nach einem der Ansprüche 8 oder 9, wobei die geschlossene Schleife in der ausgebreiteten Konfiguration annähernd senkrecht zu dem länglichen Körper (402) angeordnet ist.

11. Vorrichtung nach einem der Ansprüche 8-10, wobei die geschlossene Schleife dazu ausgestaltet ist, sich in Reaktion auf Freigabe der Zusammenziehung selbst in die ausgebreitete Konfiguration auszubreiten.

12. Vorrichtung nach einem der Ansprüche 8-11, ferner umfassend einen Speicherdraht (428), angeordnet innerhalb des Lumens der geschlossenen Schleife, wobei der Speicherdraht (428) dazu ausgestaltet ist, die geschlossene Schleife in der im Wesentlichen kreisrunden Form zu halten.

13. Vorrichtung nach einem der Ansprüche 8-12, wobei der Betätigungsdraht ferner dazu ausgestaltet ist, den länglichen Körper (402) des Katheters (400) in Reaktion auf Spannen des Betätigungsdrahts zu lenken.

14. Vorrichtung nach Anspruch 13, wobei die geschlossene Schleife dazu ausgestaltet ist, in Reaktion auf Spannen des Betätigungsdrahts in Bezug auf den länglichen Körper (402) die annähernd senkrechte Anordnung beizubehalten.

15. Vorrichtung nach einem der Ansprüche 1-14, wobei eine oder mehrere Elektrodenstrukturen (416a-k) eine Vielzahl von Elektrodenstrukturen umfasst und die Vielzahl von Elektrodenstrukturen gleichförmig um die geschlossene Schleife verteilt sind.

## Revendications

1. Appareil pour réaliser au moins une fonction prise parmi des fonctions de cartographie et d'ablation, l'appareil comprenant :
un cathéter (400) qui est dimensionné et conformé pour un accès vasculaire et qui inclut un corps allongé (402) qui s'étend entre une extrémité proximale et une extrémité distale ;
une section de pointe (406) qui est agencée au niveau de l'extrémité distale du corps allongé (402) et qui peut être déployée pour former une boucle fermée, la boucle fermée comprend une surface intérieure (412) et une surface extérieure (414), dans lequel la surface extérieure (414) comprend une surface incurvée ; et
une ou plusieurs structure(s) d'électrode(s) (416a-k) qui est/sont agencée(s) avec la section de pointe (406) ; **caractérisé en ce que** la surface intérieure (412) comprend une surface qui définit un espace cylindrique intérieur avec un axe qui est perpendiculaire au plan de la boucle fermée.

2. Appareil selon la revendication 1, dans lequel au moins une surface prise parmi la surface intérieure (412) et la surface extérieure (414) est continue le long de la boucle fermée.

3. Appareil selon la revendication 2, dans lequel les une ou plusieurs structures d'électrode(s) (416a-k) incluent une pluralité de structures d'électrode(s), et les structures d'électrode(s) de la pluralité de structures d'électrode(s) sont agencées sur la surface extérieure (414) de la boucle fermée.

4. Appareil selon la revendication 3, dans lequel chacune de la pluralité de structures d'électrode(s) (416a-k) comprend une partie incurvée.

5. Appareil selon la revendication 4, dans lequel une partie incurvée de chacune de la pluralité de structures d'électrode(s) (416a-k) est agencée sur la surface incurvée de la boucle fermée.

6. Appareil selon l'une quelconque des revendications 1 à 5, dans lequel la boucle fermée comprend une forme sensiblement circulaire.

7. Appareil selon la revendication 6, dans lequel la boucle fermée inclut une configuration déployée et une configuration restreinte, et la boucle fermée est configurée pour être réduite à la configuration restreinte en réponse à sa restriction.

8. Appareil selon la revendication 7, comprenant en outre un fil d'actionnement (422) qui est agencé à l'intérieur d'une lumière de la boucle fermée, le fil d'actionnement (422) étant configuré pour actionner la boucle fermée entre la configuration déployée et la configuration restreinte en réponse à la mise sous tension du fil d'actionnement (422).

9. Appareil selon la revendication 8, dans lequel la boucle fermée est configurée de manière à ce qu'elle conserve la forme sensiblement circulaire après qu'elle a été actionnée depuis la configuration restreinte jusqu'à la configuration déployée.

10. Appareil selon l'une quelconque des revendications 8 ou 9, dans lequel la boucle fermée est agencée approximativement perpendiculairement au corps allongé (402) dans la configuration déployée.

11. Appareil selon l'une quelconque des revendications 8 à 10, dans lequel la boucle fermée est configurée de telle sorte qu'elle réalise un auto-déploiement selon la configuration déployée en réponse à la libération de la restriction.

12. Appareil selon l'une quelconque des revendications 8 à 11, comprenant en outre un fil de mémoire (428) qui est agencé à l'intérieur de la lumière de la boucle fermée, le fil de mémoire (428) étant configuré pour conserver la boucle fermée selon la forme sensiblement circulaire.

13. Appareil selon l'une quelconque des revendications 8 à 12, dans lequel le fil d'actionnement est en outre configuré pour diriger et orienter le corps allongé (402) du cathéter (400) en réponse à la mise sous tension du fil d'actionnement.

14. Appareil selon la revendication 13, dans lequel la boucle fermée est configurée de telle sorte qu'elle maintienne l'agencement approximativement perpendiculaire par rapport au corps allongé (402) en réponse à la mise sous tension du fil d'actionnement.

15. Appareil selon l'une quelconque des revendications 1 à 14, dans lequel les une ou plusieurs structures d'électrode(s) (416a-k) comprennent une pluralité de structures d'électrode(s), et les structures d'électrode(s) de la pluralité de structures d'électrode(s) sont distribuées de façon uniforme le long de la boucle fermée.
